# EUROPEAN PATENT SPECIFICATION

(11) **EP 3 295 993 B1**
(45) Date of publication and mention of the grant of the patent: **16.10.2019**
(21) Application number: 16724923.4
(22) Date of filing: 12.05.2016
(51) Int. Cl.: A61Q 19/08, A61K 8/04, A61M 11/04, B05B 7/16, B05B 7/22, B05B 7/24, A61F 7/00, A61F 7/02

(54) **DEVICE FOR THE ADMINISTRATION OF A COSMETIC PRODUCT, CORRESPONDING PROCESS AND CONTAINER**
VORRICHTUNG ZUR VERABREICHUNG EINES KOSMETISCHEN PRODUKTS, ZUGEHÖRIGES VERFAHREN UND BEHÄLTER
DISPOSITIF POUR L'ADMINISTRATION D'UN PRODUIT COSMÉTIQUE, PROCÉDÉ ET CONTENEURS CORRESPONDANTS

(30) Priority: 15.05.2015 ES 201530571 U; 15.05.2015 ES 201530667
(43) Date of publication of application: 21.03.2018
(73) Proprietor: Beautygun S.L., 08172 Sant Cugat del Vallès (Barcelona) (ES)
(72) Inventor: PLAN, Philippe, 08172 Sant Cugat del Vallès (Barcelona) (ES)
(74) Representative: Curell Suñol S.L.P.
(86) International application number: PCT/ES2016/070361
(87) International publication number: WO 2016/185067

(56) References cited:
- WO-A1-2012/144990
- DE-A1- 3 108 918
- US-A- 6 141 985
- US-A1- 2004 102 768
- US-A1- 2011 060 195
- DATABASE WPI Week 201417 30 April 2014 (2014-04-30) Thomson Scientific, London, GB; AN 2014-E12182 XP002759334, -& JP 2014 039916 A (MTG KK) 6 March 2014 (2014-03-06)

## Description

### Field of the Invention

The invention is comprised in the field of cosmetic treatment for smoothing out wrinkles on skin.

More specifically, the invention relates to a device for the administration of a cosmetic product on the skin of a patient, generally, although not exclusively, in the facial area.

The invention also relates to the corresponding process, use and container.

### State of the Art

Treatments dedicated to smoothing out wrinkles, particularly facial wrinkles, are common in the field of anti-aging cosmetic treatments.

There are treatments based on injecting components, such as botulinum toxin type A, that seek to prevent muscle contraction, as well as other treatments based on injecting components, such as collagen, that can be used to fill wrinkled areas. All these have different degrees of effectiveness but have the drawbacks that they are unpleasant for the user, not without certain risks and must be administered by qualified medical staff.

On the other hand, there are many cosmetic products for topical application, such as creams, balms, or the like. They are generally applied on the skin of the patient by the person performing the cosmetic treatment or even by patients themselves using their hands.

However, the effectiveness of said products is hampered by reduced absorption due to this method of administration, part of the product remaining on the hands of the person applying the treatment and saturating the skin such that part of the product never penetrates the skin. That is particularly significant for customers in the case of expensive products, in which there may be a large amount of product that is not used in its entirety.

For this reason, a method of application which allows a wrinkle-smoothing effect, which is easy to administer and can be used by people without medical training is necessary.

US 2011/060195 A1 relates to a cosmetic treatment method that treats the human scalp, including applying a cosmetic composition and/or a gas to the scalp with a spray device operating with a compressed gas, a mean size of the sprayed particles of composition lying in the range 10 am to 35 µm.

WO 2012/1144990 A1 discloses a noninvasive transdermal delivery device that relates generally to a handheld mechanical apparatus for noninvasive transdermal administration of gas, small to large water- soluble (hydrophilic) pharmaceutical agents, vitamins, and other therapeutic agents. Components of such delivery devices, methods of producing a substance comprising a supersaturated amount of a dissolved gas, as well as, methods of administering a therapeutic agent using such delivery devices and methods of treating a disease or condition using such delivery devices are also disclosed.

### Description of the Invention

The purpose of the invention is to provide a device for the administration of a cosmetic product, of the type indicated above, which allows favoring the absorption and effectiveness of said cosmetic product.

This purpose is achieved by means of a device for the administration of a product of the type indicated above, characterized in that it comprises a first holder for a pressurized gas cartridge, a second holder for a cosmetic product container, flow rate regulating means, an outlet nozzle, heating means of said nozzle, and a trigger. The first holder is configured for housing a pressurized gas cartridge for the passage of a pressurized gas towards the flow rate regulating means, the first holder, the flow rate regulating means and the nozzle being fluidically connected, such that the regulating means are located downstream of the first holder and upstream of the outlet nozzle. The second holder is configured for housing a cosmetic product container for the passage of a cosmetic product towards the nozzle, and the second holder being fluidically connected with the nozzle by means of a first product outlet conduit. The trigger is configured for opening the passage of the gas when it is operated by a user.

The pressurized gas is therefore mixed with the cosmetic product in the nozzle, generating an aerosol that exits through said nozzle and reaches the skin of the patient with a certain pressure still. The effect of this outlet pressure makes it easier for the product to reach the skin and for it to be absorbed. Furthermore, as the aerosol exits the nozzle the change in pressure causes the cooling of said aerosol. It has been experimentally proven that the low temperature of the aerosol as it reaches the skin combined with the pressurized spraying creates a synergy that is particularly advantageous for the cosmetological reduction of facial wrinkles, while at the same time maintaining ease of use. Using disposable components such as pressurized gas cartridges or the product container allows not having to connect the device for it to work, making the handling thereof easier. Given that common cartridges for pressurized gases usually have an excessively high pressure for direct application, the device is provided with regulating means reducing the gas outlet pressure. Similarly, given that the change in pressure at the outlet of the nozzle might cause it to cool excessively, freezing the product and plugging the nozzle, the heating means of the nozzle have the purpose of heating said nozzle so that this does not happen. The device thus created can be handled manually, with a simple method of application, and the customers can furthermore observe an added value with respect to a normal topical treatment, which confers airs of prestige to the device, assuring the differentiation thereof from a normal topical treatment, which is also an advantage for the highly competitive sector of cosmetic treatments.

Several preferred embodiments the features of which are described in the dependent claims have been provided based on the invention defined in the main claims.

The outlet nozzle is preferably made of a ceramic material. It can therefore withstand low temperatures, as well as sudden changes which may occur, and can be heated easily at the same time, applying a resistance, for example.

In an advantageous embodiment, the first product outlet conduit has an inner diameter comprised in the range of between 0.4 mm and 1.2 mm, preferably between 0.5 mm and 1 mm, more preferably 0.7 mm, depending on the fluidity of the cosmetic product and on the angle of inclination of said conduit, being narrower with respect to higher fluidity and more vertical. The diameter must be a compromise which prevents product loss due to dripping when there is no passage of gas and which at the same time allows the product to exit. This first outlet conduit is preferably connected to a first needle located in said second holder suitable for perforating the cover (made of elastomer, for example) for closing the cosmetic product container.

In an advantageous embodiment, the device of the invention also comprises first securing means for securing said pressurized gas cartridge against said flow rate regulating means, configured such that the attachment between an outlet of said pressurized gas cartridge and an inlet of said flow rate regulating means is sealed. By maintaining a firm and sealed attachment gas leaks are prevented, causing a more homogenous operation of the device and with less noise, furthermore being able to better use the gas contained in the cartridge.

In an alternative embodiment, the flow rate regulating means and the second holder are furthermore fluidically connected by means of a second conduit which extending to the second holder and preferably connected to a second needle located in the second holder configured for perforating the cover of the container. This second inlet conduit allows a part of the gas to be diverted into the cosmetic product container and force said product to exit through the first outlet conduit towards the nozzle. This facilitates a uniform operation and prevents interruptions in product outflow as long as there is product remaining in the container.

Advantageously, said second gas inlet conduit has an inner diameter comprised in the range of between 0.4 mm and 1.2 mm, preferably between 0.5 mm and 1 mm, more preferably 0.7 mm. The diameter of this conduit together with the gas pressure at the point where it is located determine the amount of gas reaching the container and pushing the product towards the first outlet conduit. The person skilled in the art will select the specific parameters according to the final configuration of the device, the information herein provided as an example is what has been observed as having a better ratio for a constant evacuation that is neither too fast nor too slow.

Preferably, the second holder further comprises second securing means for said cosmetic product container, such that the container is secured without it becoming detached. Furthermore, in configurations having a second gas inlet conduit for the entry of gas into the container, this fixing prevents the container from popping out due to the effect of pressure.

In an advantageous embodiment, the cosmetic product container contains a mixture of a cosmetic product with an alcohol. The viscosity of the liquid can therefore be adapted to the suitable value so that the liquid can effortlessly exit towards the nozzle and not drip at the same time.

Advantageously, the amount of said alcohol is in the range of between 0% and 25% with respect to the total volume, preferably between 5% and 20%, more preferably between 10% and 15%. It has been experimentally proven that for the desired cosmetic products, this is the percentage offering the best viscosity ratio without becoming unpleasant for the patient who may refuse the administration of a product with an excessive alcohol content.

In an advantageous embodiment, the heating means are configured for heating the nozzle to an initial temperature in the range comprised between 60°C and 100°C, preferably between 70°C and 90°C, more preferably 85°C. Initial temperature is understood as the temperature it is at when the device is ready for use, and final temperature is understood as the temperature it is at when the established treatment time ends. These are the values obtained by means of experiments allowing a sufficiently cold exit of the aerosol without the product freezing in the nozzle.

The heating means are preferably configured for heating the nozzle to said initial temperature in a time less than 10 seconds, preferably less than 7 seconds, more preferably less than 5 seconds. These are reasonable values that can lead a user to expect that the device is ready to start applying the treatment on the patient. Longer times give the users and patients the feeling that the device is one of low quality.

In an advantageous embodiment, the first holder is configured for housing a cartridge containing CO₂ at a pressure of 50 bar.

In an advantageous embodiment, the second holder is configured for housing a container containing an amount of product which is in the range of 2 to 6 cm³, preferably 3 to 5 cm³, more preferably 4 cm³. Smaller sizes may be too limited for a complete treatment, whereas larger sizes may cause excessive product wastage.

In an advantageous embodiment, the device is configured such that the time of evacuating said pressurized gas cartridge is in the range of 2 to 10 minutes, preferably 3 to 6 minutes, more preferably 4 minutes. These values are obtained experimentally for the optimum duration of treatment. Lower values may be insufficient for a complete treatment, whereas higher values can irritate patients or their skin.

The device of the invention is preferably configured such that for the time of evacuating the pressurized gas cartridge, cosmetic product consumption is in the range of 1 to 10 cm³, preferably of 1 to 5 cm³, more preferably 3 cm³. These are advantageous values for treatment given the area to be covered and the application time; a smaller amount would not be sufficient for treatment, whereas larger amounts would lead to excessive product wastage.

In an alternative embodiment, the device further comprises power supply means. It can therefore be provided with electronic control, indication and heating systems.

Preferably, it further comprises a voltage regulator such that the voltage from the power supply is adapted to the operating parameters required by the electrical and electronic components of the device.

In an advantageous embodiment, the power supply means comprise a battery which allows using the device without cables. Said battery can be rechargeable, and, optionally, there is a support and charging base where the user places the device between uses.

Said battery preferably has a voltage in the range comprised between 9 V and 15 V, preferably between 10 V and 12 V, more preferably 11 V. These are voltage ranges that can be obtained on the market for batteries of a suitable size to be placed in the device.

The voltage regulator preferably comprises a voltage booster given that some of the components may require operating at a voltage higher than that offered by the battery.

Said voltage booster is preferably configured for providing an output voltage comprised in the range of 22 V to 26 V, preferably 24 V. These are suitable voltage ranges for some flow rate regulating components.

In a preferred embodiment, the flow rate regulating means comprise an injector. Such components, which are common in the automotive sector, have the advantage of a simple regulation possibility together with high reliability, and a low enough consumption ratio for use thereof in a manual device, making it particularly suitable.

The injector works cyclically at a specific frequency. Within each cycle, it remains open for a certain time and closed the rest of the time of each cycle. In this context, working cycle refers to the time ratio between the injector being open and closed per cycle, generally being expressed as a percentage of time it remains open with respect to the total time. The working frequency of said injector is preferably 60 Hz which is within the working range of the injector. Lower frequencies would make the proper working thereof difficult, whereas higher frequencies may become unpleasant for the user, and even convey a feeling of the product being unreliable.

The injector is preferably configured for having a working cycle comprised between 1% and 15%, preferably between 3% and the 10%, more preferably between 5% and 8%. Experimentally, these are the most suitable ranges and values for regulating the outlet pressure of the gas cartridge given the gas pressure values said cartridges usually have.

In an advantageous embodiment, the device further comprises a control module configured for regulating flow rate regulating parameters of the flow rate regulating means, and temperature regulating parameters of the heating means of the nozzle. The regulation of the flow rate exiting the pressurized gas cartridge can therefore be adapted in order to try to obtain a uniform flow rate at the outlet of the nozzle as said cartridge is gradually evacuated. Similarly, as pressure gradually decreases, the nozzle needs less heating to prevent it from freezing, at the same time preventing excessive heating that would heat the outgoing mixture of product and gas excessively, losing the desired cooling effect.

In an advantageous embodiment, the present invention further comprises a display screen in communication with the control module and configured for receiving a plurality of data from the control module and displaying said plurality of data. This screen can be used, for example and not exclusively, so that the user can see the remaining application time, the presence or absence of a gas cartridge or a product container, the initial temperature indication, etc.

In an advantageous embodiment, the present invention further comprises an audible indicator device. It is a simple system for indicating the key operating moments to the user, such as the fact that the device is ready for use or that the application time has ended. The user can thus operate normally keeping their attention fixed only on the patient.

In an advantageous embodiment, the flow rate regulating means comprise an injector and the flow rate regulating parameters comprise a value of the working cycle of said injector.

The control module is preferably configured for varying said value of the working cycle of the injector between an initial value of 5% to a final value of 8% such that for each cycle, the passage of gas opens for a shorter time at the beginning than at the end, thereby compensating for the pressure decline of the cartridge as said cartridge is gradually evacuated.

In an advantageous embodiment, the temperature regulating parameters are suitable for regulating an initial temperature in the range comprised between 60°C to 100°C, preferably between 70°C to 90°C, more preferably 85°C, and a final temperature in the range comprised between 0°C and 20°C, preferably between 5°C and 15°C, more preferably 10°C. This initial temperature must compensate for the sudden cooling of the nozzle when the pressurized gas starts to exit, and maintain a temperature above the point at which the product would freeze, blocking the outlet nozzle.

In an alternative embodiment, the present invention further comprises a battery and a voltage booster, and the control module is furthermore configured for regulating voltage boosting parameters of said voltage booster. The use of a battery confers autonomy and allows using the device without cables; on the other hand, given that most batteries have a reduced voltage, there is a need to boost said voltage so that it is capable of powering some of the components, for example, the flow rate regulating means.

The voltage boosting parameters preferably follow a function that increases over time. Given that the battery gradually loses voltage as it loses its charge, the voltage booster gradually compensates for said drop such that the final voltage is maintained within the operating range.

In a preferred embodiment, the device of the invention has a general gun shape which makes it easier to handle, the trigger being suitably placed for being operated with the index finger, and the nozzle being located in the front part. The device preferably has a base serving both for supporting same and for performing the functions of a battery charging station.

The invention therefore allows a process for the administration of a cosmetic product which, based on a device such as the one that has been described, comprises the steps of:
[a] a user placing a pressurized gas cartridge in the first holder and a cosmetic product container in the second holder,
[b] the user actuating the trigger,
[c] the heating means heating the nozzle,
[d] the trigger opening the passage of a pressurized gas located inside the pressurized gas cartridge,
[e] the flow rate regulating means regulating the outflow rate of the gas through said nozzle,
[f] as it exits, said gas also causing a cosmetic product located inside the cosmetic product container to exit, and
[g] the cosmetic product being mixed with the gas in said nozzle and sprayed on the skin of a patient.

Accordingly, it is a simplified process based on easily replaceable consumables which allows spraying a cosmetic product on the skin with a certain pressure and at a low temperature, with the benefits that have been described above.

In an advantageous embodiment, the present invention further comprises a prior step consisting of said heating means heating said nozzle in a moment before step [c] such that the burst of cold produced when the gas starts to exit through the nozzle is compensated for without the product becoming frozen and the outlet being blocked.

Preferably, in the moment in which the gas starts to exit through the nozzle, the heating means have heated the nozzle to an initial temperature in the range comprised between 60°C and 100°C, preferably between 70°C and 90°C, more preferably 85°C, as described above.

The heating means preferably heat the nozzle to the initial temperature in a time less than 10 seconds, preferably less than 7 seconds, more preferably less than 5 seconds, such that the initial user wait time is short.

In an alternative embodiment, when the nozzle reaches the initial temperature the device emits an indication in the form of an acoustic signal, a visual signal or a combination of both. This therefore allows indicating to the user that the device is ready for use.

In an advantageous embodiment, a part of the pressurized gas is introduced in the product container through a second gas inlet conduit, and it forces said product to exit through the first product outlet conduit such that it helps in the evacuation and prevents interruptions in product outflow towards the nozzle.

In an alternative embodiment, a control module provided in the device controls the flow rate regulating parameters of the flow rate regulating means and temperature regulating parameters of the heating means of the nozzle, as described above.

In an advantageous embodiment, the flow rate regulating means comprise an injector and the flow rate regulating parameters comprise a working cycle of said injector, as described above.

The working cycle of said injector is preferably varied in a range comprised between 1% and 15%, preferably between 3% and 10%, more preferably between 5% and 8%, compensating for the drop in gas pressure as the pressurized gas cartridge is evacuated.

In an advantageous embodiment, said temperature regulating parameters are varied such that the temperature of said nozzle goes from an initial temperature in the range comprised between 60°C to 100°C, preferably between 70°C to 90°C, more preferably 85°C, to a final temperature in the range comprised between 0°C and 20°C, preferably between 5°C and 15°C, more preferably 10°C, allowing an initial temperature preventing the nozzle from being blocked due to freezing of the product and maintaining a cold temperature during the process but preventing said freezing.

In another alternative embodiment, the device further comprises a battery and a voltage booster, and the control module further regulates voltage boosting parameters of said voltage booster, which allows using the device without cables and adapting the voltage to the requirements of the different components, as described above.

The control module preferably increases the voltage boosting parameters following a function that increases over time, compensating for the possible voltage drop in the battery as it loses its charge.

The invention therefore uses a pressurized gas for spraying a cosmetic product on the skin of a patient through a nozzle of a device, in which the outlet temperature is regulated at least at a minimum level so that ice is not formed on said nozzle or so that said ice does not end up blocking the passage of said cosmetic product through said nozzle.

Likewise, the invention describes a cosmetic product container suitable for use thereof in a device such as the one described, comprising a main body and a cover, made of elastomer, for example, said container preferably being a vial, and containing a mixture of cosmetic product with an alcohol.

Said cosmetic product preferably contains an anti-wrinkle and/or anti-aging agent which, along with the method of application, has the advantages that have been described above.

In an advantageous embodiment, the amount of said alcohol is in the range of between 0% and 25% with respect to the total volume, preferably between 5% and 20%, more preferably between 10% and 15%.

In an advantageous embodiment, said container contains an amount of product which is in the range of 2 to 6 cm³, preferably 3 to 5 cm³, more preferably 4 cm³, such that it allows, with the device that has been described, the required range of application time, maintaining consumption with the pre-established ranges.

The invention also comprises other detailed features illustrated in the detailed description of an embodiment of the invention and in the attached drawings.

### Brief Description of the Drawings

The advantages and features of the invention are seen based on the following description in which a preferred embodiment of the invention is described in a non-limiting manner with respect to the scope of the main claim in reference to the drawings.
Figure 1 is a perspective view of the device of the invention.
Figure 2 is a simplified section view of the device
Figure 3 is a simplified perspective view of a part of the gas circuit of the device.
Figure 4 is a detailed perspective view of the nozzle

### Detailed Description of an Embodiment of the Invention

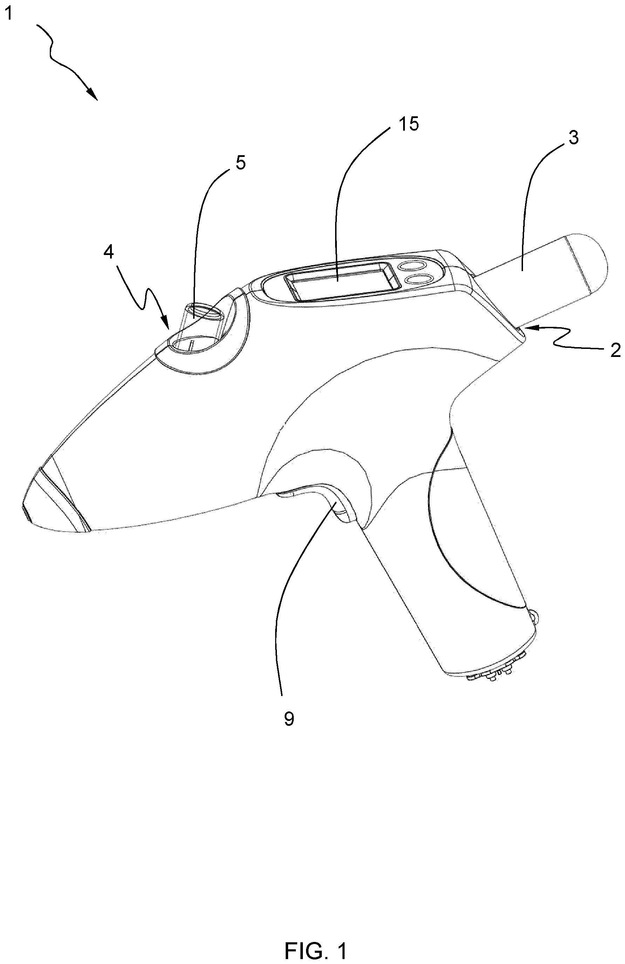

The embodiment shown in Figures 1 to 4 is a device 1 with a general gun shape, such that the user can hold it by the handle and pull the trigger 9 with the index finger.

Figure 1 shows the pressurized gas cartridge 3 being installed in the first holder 2 and the product container 5 in the second holder 4, as well as a display screen 15 to provide indications to the user. Some controls for operations such as turning the device 1 on and off can also be seen next to the screen 15. For the exemplifying embodiment, the cartridge contains CO₂ at a pressure of 50 bar, and the product container is a vial with a cover made of elastomer containing 4 cm³ of a mixture of a cosmetic product with 10% to 15% alcohol. The percentage has been chosen for obtaining the desired viscosity given the rest of the components, as will be described below.

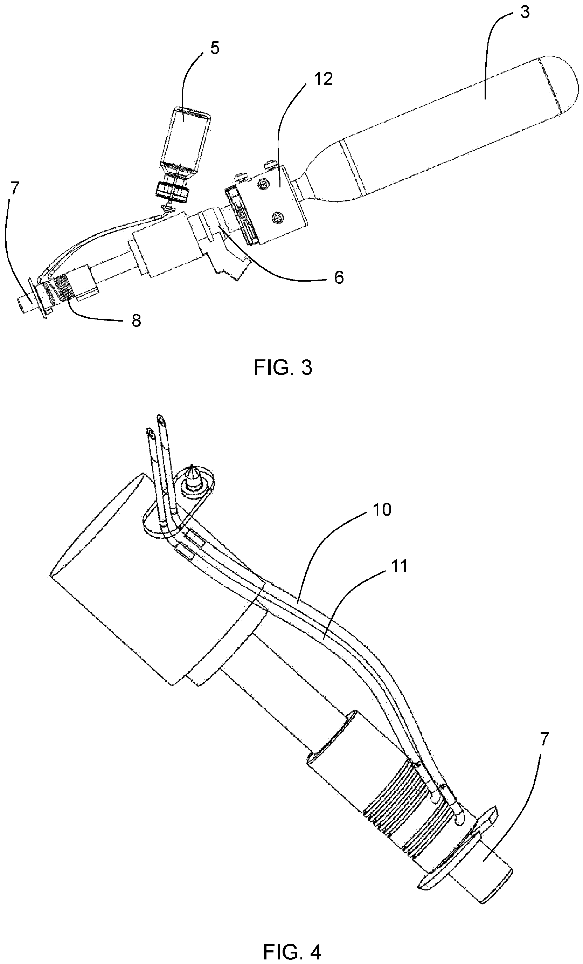

The exemplifying embodiment shows a preferred configuration in Figure 3, in which the axis of the product container can be seen forming an acute angle with respect to the axis of the nozzle, to make product outflow easier.

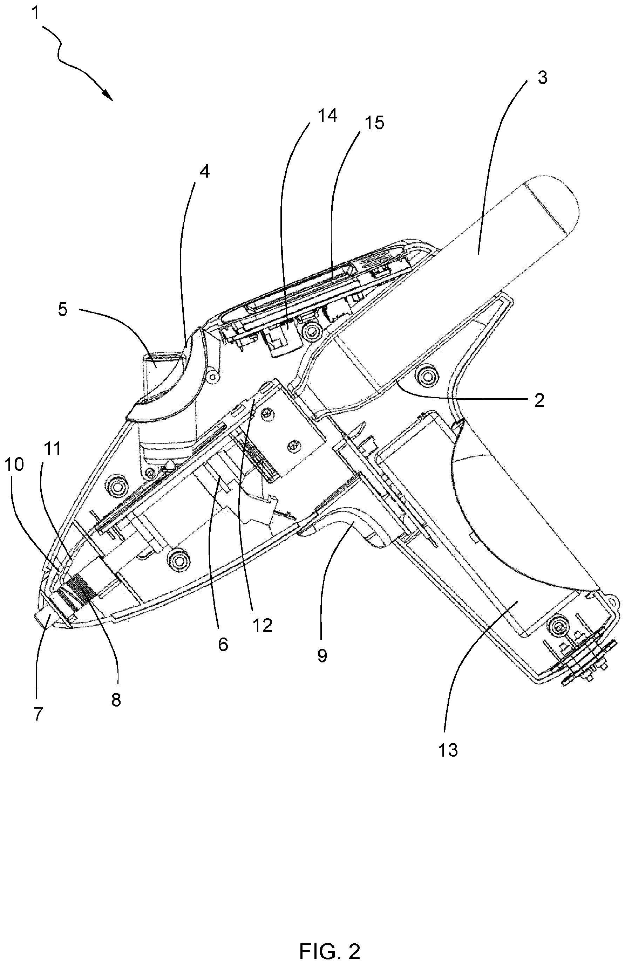

In the embodiment herein described, the device 1 does not need cables for operation, given that the power supply means 13 comprise a rechargeable 11 V battery, which can be seen in Figure 2. Although not shown in the drawings, the device 1 has a storage and charging base for said battery.

Figure 2 shows a simplified section view of the exemplifying embodiment, in which most of the inner wiring or other support elements are not shown for the sake of clarity. Said Figure 2 shows how the cartridge 3 is placed in the holder and is secured by securing means 12 keeping it firmly secured to the flow rate regulating means 6 to cause sealing which prevents unwanted gas leaks. In the particular case of the example, the flow rate regulating means 12 comprise an injector of the type used in vehicles, which is operated at a frequency of 60 Hz and with working cycles varying between 5% and 8%. Given that the operating voltage of the injector used in the example is 24 V, the device also includes voltage regulating means for increasing the rated voltage of the battery from 11 V to 24 V as required by the injector. Given that the voltage decreases as the battery loses its charge, the regulator follows a profile that increase over time to keep the voltage level within the operating ranges of the injector. The voltage regulating parameters are controlled by means of a control module 14.

When the pressurized gas from the cartridge 3 goes through the injector, its pressure is reduced such that the pressure is no longer too high when the gas reaches the nozzle 7. Part of this gas is introduced through a second gas inlet conduit 11 in the container 5 and it helps the product to exit through the first outlet conduit 10, thereby obtaining an aerosol mixture of pressurized gas and cosmetic product which is discharged through the nozzle 7 for application thereof. Likewise, at the moment of exiting through the nozzle 7, the sudden change in pressure causes a drop in temperature and the combined cosmetic effect of applying sprayed product under pressure and at a low temperature occurs, which favors smoothing out the wrinkles on the patient. For the case of the example, the conduits 10 and 11 have an inner diameter of 0.7 mm which, in combination with the viscosity of the product, allows the product to exit smoothly without dripping when it is not being applied. The detail of Figure 4 shows both conduits 10 and 11 entering the container 5 through needles penetrating the cover made of elastomer.

In the example, to prevent the container from popping out due to the incoming gas, the second holder 4 is provided with indentations which act like securing means. They are not shown in the drawings for the sake of simplicity.

Likewise, in the embodiment the nozzle 7 is formed from a ceramic material so that it can withstand temperature changes. The device has electric cables wound on the nozzle which act like heating means 8 by way of resistance that is heated when current circulates. The nozzle 7 of the example has grooves keeping said cables in place, which can be seen in Figure 4. In the example, an initial temperature of 85°C has been determined, the nozzle having to reach said temperature before the gas starts to circulate to thereby prevent the cosmetic product from freezing, which could block the outlet. The users thereby activate the device 1, heating the nozzle 7 for a maximum of 5 seconds, and said users will be notified of the moment in which they can begin to apply the treatment on the patient by means of the display screen 15 and an acoustic indicator device.

The sizes and flow rates of the example have been selected to enable providing a 4-minute treatment time, after which the cartridge 3 no longer has enough gas to maintain the pressure, and the container 5 does not contain enough product for it to continue being evacuated, specifically the residual product volume of the example is around 1 cm³, so 3 cm³ are consumed during application.

In the exemplifying embodiment, when the device 1 starts operating, the control module 14 acts to increase the working cycle of the injector from an initial value of 5% to a final value of 8% in order to compensate for the drop in gas pressure as the cartridge 3 is evacuated. Likewise, the control module 14 also sends an electric current to the heating means 8 of the nozzle 7 in order to reach the 85°C which have been described initially; as time passes and the gas pressure decreases, the cooling of the nozzle is reduced so, along with the thermal inertia of the ceramic material of the nozzle 7, so much heating is not needed, and the heating means can even be disconnected, such that it is kept at a temperature of about 10°C.

In the exemplifying embodiment, in order to reduce the final product cost, the specific temperature and flow rate regulation profile has been obtained experimentally and has been pre-programmed into the memory of the control module 14 such that on-the-go calculations and the electrical consumption of said module 14 are prevented. The person skilled in the art will understand that there may be other configurations based, for example, on sensors and on a compensation adjusted to environmental conditions.

The person skilled in the art will also understand that there are other possible variations of the invention without departing from the scope of the main claim as a result. For example, though in a non-exclusive manner, the sizes and content of the container and cartridge, the flow rate regulating means and the operating parameters thereof, as well as the shapes and relative placement of each of the auxiliary elements, may vary.

## Claims

1. A device (1) for the administration of a cosmetic product, **characterized in that** it comprises:
- a first holder (2) for a pressurized gas cartridge (3),
- a second holder (4) for a cosmetic product container (5),
- flow rate regulating means (6),
- an outlet nozzle (7),
- heating means (8) of said nozzle (7),
- a trigger (9), and
- a control module (14),
wherein said first holder (2) is configured for housing a pressurized gas cartridge (3) for the passage of a pressurized gas towards said flow rate regulating means (6), said first holder (2), said flow rate regulating means (6) and said nozzle (7) being fluidically connected, such that said regulating means are located downstream of said first holder (2) and upstream of said outlet nozzle (7),
wherein said second holder (4) is configured for housing a cosmetic product container (5) for the passage of a cosmetic product towards said nozzle (7), and said second holder (4) being fluidically connected with said nozzle (7) by means of a first product outlet conduit (10),
and wherein said trigger (9) is configured for opening the passage of said gas when it is operated by a user, and said control module (14) is configured for regulating flow rate regulating parameters of said flow rate regulating means (6), and temperature regulating parameters of said heating means (8) of said nozzle (7).

2. The device (1) according to claim 1, **characterized in that** said outlet nozzle (7) is made of a ceramic material.

3. The device (1) according to any of claims 1 or 2, **characterized in that** it also comprises first securing means (12) for securing said pressurized gas cartridge (3) against said flow rate regulating means (6), configured such that the attachment between an outlet of said pressurized gas cartridge (3) and an inlet of said flow rate regulating means (6) is sealed.

4. The device (1) according to any of claims 1 to 3, **characterized in that** said flow rate regulating means (6) and said second holder (4) are furthermore fluidically connected by means of a second conduit (11) extending to said second holder (4); and preferably wherein any of said first product outlet conduit (10) or said second gas inlet conduit (11) has an inner diameter comprised in the range of between 0.4 mm and 1.2 mm, preferably between 0.5 mm and 1 mm, more preferably 0.7 mm.

5. The device (1) according to any of claims 1 to 4, **characterized in that** said second holder (4) further comprises second securing means (12) for said cosmetic product container (5).

6. The device (1) according to any of claims 1 to 5, **characterized in that** said first holder (2) is configured for housing a cartridge (3) containing CO₂ at a pressure of 50 bar.

7. The device (1) according to any of claims 1 to 6, **characterized in that** said second holder (4) is configured for housing a container (5) containing an amount of product which is in the range of 2 to 6 cm³, preferably 3 to 5 cm³, more preferably 4 cm³.

8. The device (1) according to any of claims 1 to 7, **characterized in that** it further comprises power supply means (13) and a voltage regulator; wherein said power supply means (13) preferably comprises a battery having a voltage in the range comprised between 9 V and 15 V, preferably between 10 V and 12 V, more preferably 11 V; and said voltage regulator preferably comprises a voltage booster configured for providing an output voltage comprised in the range of 22 V to 26 V, preferably 24 V.

9. The device (1) according to any of the claims 1 to 8, **characterized in that** said flow rate regulating means (6) comprise an injector, and said flow rate regulating parameters comprise a value of the working cycle of said injector, said working cycle being defined as the ratio between the time in which said injector is open with respect to the total time.

10. A process for the administration of a cosmetic product which, starting from a device (1) according to claim 1, comprises the steps of:
[a] a user placing a pressurized gas cartridge (3) in said first holder (2) and a cosmetic product container (5) in said second holder (4),
[b] the user actuating said trigger (9),
[c] said heating means (8) heating said nozzle (7),
[d] said trigger (9) opening the passage of a pressurized gas located inside said pressurized gas cartridge (3),
[e] said flow rate regulating means (6) regulating the outflow rate of the gas through said nozzle (7),
[f] as it exits, said gas also causing a cosmetic product located inside said cosmetic product container (5) to exit, and
[g] said cosmetic product being mixed with the gas in said nozzle (7) and sprayed on the skin of a patient;
wherein said control module (14) provided in said device (1) controls the flow rate regulating parameters of said flow rate regulating means (6) and temperature regulating parameters of said heating means (8) of the nozzle (7).

11. The process according to claim 10, **characterized in that** it further comprises a prior step consisting of said heating means (8) heating said nozzle (7) in a moment before step [c].

12. The process according to claim 11, **characterized in that** in the moment in which the gas starts to exit through said nozzle (7), said heating means (8) have heated said nozzle (7) to an initial temperature in the range comprised between 60°C and 100°C, preferably between 70°C and 90°C, more preferably 85°C.

13. The process according to any of claims 10 to 12, **characterized in that** a part of said pressurized gas is introduced in said product container (5) through a second gas inlet conduit (11) and forces said product to exit through said first product outlet conduit (10).

14. The process according to any of the claims 10 to 13, **characterized in that** said flow rate regulating means (6) comprise an injector and said flow rate regulating parameters comprise a working cycle of said injector, said working cycle being defined as the ratio between the time in which said injector is open with respect to the total time.

15. The process according to claim 14, **characterized in that** said working cycle of said injector is varied in a range comprised between 1% and 15%, preferably between 3% and 10%, more preferably between 5% and 8%.

16. The process according to any of claims 10 to 15, **characterized in that** said temperature regulating parameters are varied such that the temperature of said nozzle (7) goes from an initial temperature in the range comprised between 60°C to 100°C, preferably between 70°C to 90°C, more preferably 85°C, to a final temperature in the range comprised between 0°C and 20°C, preferably between 5°C and 15°C, more preferably 10°C.

17. The process according to any of claims 10 to 16, **characterized in that** said device (1) further comprises a battery and a voltage booster, and said control module (14) further regulates voltage boosting parameters of said voltage booster by increasing said voltage boosting parameters following a function that increases over time.

18. A cosmetic product container (5) suitable for use thereof in a device (1) according to claim 1, comprising a main body and a cover, made of elastomer, for example, said container (5) preferably being a vial, and containing a mixture of cosmetic product with an alcohol.

19. The cosmetic product container (5) according to claim 18, **characterized in that** the amount of said alcohol is less than 25% with respect to the total volume, preferably between 5% and 20%, more preferably between 10% and 15%.

## Patentansprüche

1. Vorrichtung (1) zur Anwendung eines Kosmetikprodukts, **dadurch gekennzeichnet, dass** sie umfasst:
- eine erste Halterung (2) für eine Druckgaskartusche (3),
- eine zweite Halterung (4) für einen Kosmetikproduktbehälter (5),
- Durchflussregulierungsmittel (6),
- eine Auslassdüse (7),
- Heizmittel (8) für die Düse (7),
- einen Auslöser (9), und
- ein Steuermodul (14),
wobei die erste Halterung (2) dazu ausgelegt ist, eine Druckgaskartusche (3) aufzunehmen, damit ein Druckgas zu den Durchflussregulierungsmitteln (6) fließt, wobei die erste Halterung (2), die Durchflussregulierungsmittel (6) und die Düse (7) fluidisch verbunden sind, sodass die Durchflussregulierungsmittel stromabwärts von der ersten Halterung (2) und stromaufwärts von der Auslassdüse (7) angeordnet sind,
wobei die zweite Halterung (4) dazu ausgelegt ist, einen Kosmetikproduktbehälter (5) aufzunehmen, damit ein Kosmetikprodukt zu der Düse (7) fließt, und wobei die zweite Halterung (4) mittels einer ersten Produktauslassleitung (10) fluidisch mit der Düse (7) verbunden ist,
und wobei der Auslöser (9) dazu ausgelegt ist, den Durchgang für das Gas zu öffnen, wenn er von einem Nutzer bedient wird, und wobei das Steuerungsmodul (14) dazu ausgelegt ist, die durchflussregulierenden Parameter der Durchflussregulierungsmittel (6) sowie die temperaturregulierenden Parameter der Heizmittel (8) der Düse (7) zu regulieren.

2. Vorrichtung (1) nach Anspruch 1, **dadurch gekennzeichnet, dass** die Auslassdüse (7) aus einem Keramikmaterial besteht.

3. Vorrichtung (1) nach einem der Ansprüche 1 oder 2, **dadurch gekennzeichnet, dass** sie außerdem erste Befestigungsmittel (12) zum Befestigen der Druckgaskartusche (3) an den Durchflussregulierungsmitteln (6) umfasst, die dazu ausgelegt sind, dass die Verbindung zwischen einem Auslass der Druckgaskartusche (3) und einem Einlass der Durchflussregulierungsmittel (6) abgedichtet ist.

4. Vorrichtung (1) nach einem der Ansprüche 1 bis 3, **dadurch gekennzeichnet, dass** die Durchflussregulierungsmittel (6) und die zweite Halterung (4) darüber hinaus mittels einer zweiten Leitung (11), die sich zu der zweiten Halterung (4) erstreckt, fluidisch verbunden sind; und vorzugsweise wobei irgendeine der ersten Produktauslassleitung (10) oder der zweiten Gaseinlassleitung (11) einen Innendurchmesser im Bereich von 0,4 mm bis 1,2 mm, vorzugsweise von 0,5 mm bis 1 mm, weiter vorzugsweise von 0,7 mm aufweist.

5. Vorrichtung (1) nach einem der Ansprüche 1 bis 4, **dadurch gekennzeichnet, dass** die zweite Halterung (4) weiterhin zweite Befestigungsmittel (12) für den Kosmetikproduktbehälter (5) umfasst.

6. Vorrichtung (1) nach einem der Ansprüche 1 bis 5, **dadurch gekennzeichnet, dass** die erste Halterung (2) dazu ausgelegt ist, eine Kartusche (3), die CO₂ mit einem Druck von 50 Bar enthält, aufzunehmen.

7. Vorrichtung (1) nach einem der Ansprüche 1 bis 6, **dadurch gekennzeichnet, dass** die zweite Halterung (4) dazu ausgelegt ist, einen Behälter (5) aufzunehmen, der eine Menge des Produkts enthält, die in dem Bereich von 2 bis 6 cm³, vorzugsweise 3 bis 5 cm³, weiter vorzugsweise bei 4 cm³ liegt.

8. Vorrichtung (1) nach einem der Ansprüche 1 bis 7, **dadurch gekennzeichnet, dass** sie weiterhin ein Stromversorgungsmittel (13) und einen Spannungsregler umfasst; wobei das Stromversorgungsmittel (13) vorzugsweise eine Batterie mit einer Spannung im Bereich von 9 V bis 15 V umfasst, vorzugsweise von 10 V bis 12 V, weiter bevorzugter 11 V; und wobei der Spannungsregler vorzugsweise einen Spannungsverstärker umfasst, der dazu ausgelegt ist, eine Ausgangsspannung im Bereich von 22 V bis 26 V bereitzustellen, vorzugsweise 24 V.

9. Vorrichtung (1) nach einem der Ansprüche 1 bis 8, **dadurch gekennzeichnet, dass** die Durchflussregulierungsmittel (6) eine Einspritzdüse umfassen, und wobei die Durchflussregulierungsparameter einen Wert des Arbeitszyklus der Einspritzdüse umfassen, wobei der Arbeitszyklus als das Verhältnis zwischen der Zeit, in der die Einspritzdüse offen ist, zu der Gesamtzeit definiert ist.

10. Verfahren zur Anwendung eines Kosmetikprodukts, das, beginnend bei einer Vorrichtung (1) nach Anspruch 1, folgende Schritte umfasst:
[a] ein Nutzer platziert eine Druckgaskartusche (3) in der ersten Halterung (2) und einen Kosmetikproduktbehälter (5) in der zweiten Halterung (4),
[b] der Nutzer bedient den Auslöser (9),
[c] die Heizmittel (8) erwärmen die Düse (7),
[d] der Auslöser (9) öffnet den Durchgang für ein Druckgas, das sich in der Druckgaskartusche (3) befindet,
[e] die Durchflussregulierungsmittel (6) regulieren die Abflussrate des Gases durch die Düse (7),
[f] während es austritt, veranlasst das Gas das Kosmetikprodukt in dem Kosmetikproduktbehälter (5) dazu, ebenfalls auszutreten, und
[g] das Kosmetikprodukt wird mit dem Gas in der Düse (7) vermischt und auf die Haut eines Patienten gesprüht;
wobei das Steuerungsmodul (14) in der Vorrichtung (1) die durchflussregulierenden Parameter der Durchflussregulierungsmittel (6) sowie die temperaturregulierenden Parameter der Heizvorrichtung (8) der Düse (7) steuert.

11. Verfahren nach Anspruch 10, **dadurch gekennzeichnet, dass** es weiterhin einen vorhergehenden Schritt umfasst, in dem die Heizmittel (8) die Düse (7) einen Moment vor Schritt [c] erwärmen.

12. Verfahren nach Anspruch 11, **dadurch gekennzeichnet, dass** in dem Moment, in dem das Gas beginnt, durch die Düse (7) auszutreten, die Heizmittel (8) die Düse (7) auf eine Anfangstemperatur im Bereich von 60 °C bis 100 °C erwärmt haben, vorzugsweise von 70 °C bis 90 °C, weiter vorzugsweise 85 °C.

13. Verfahren nach einem der Ansprüche 10 bis 12, **dadurch gekennzeichnet, dass** ein Teil des Druckgases durch eine zweite Gaseinlassleitung (11) in den Produktbehälter (5) eingebracht wird und das Produkt dazu zwingt, durch die erste Produktauslassleitung (10) auszutreten.

14. Verfahren nach einem der Ansprüche 10 bis 13, **dadurch gekennzeichnet, dass** die Durchflussregulierungsmittel (6) eine Einspritzdüse umfassen, und wobei die Durchflussregulierungsparameter einen Arbeitszyklus der Einspritzdüse umfassen, wobei der Arbeitszyklus als das Verhältnis von der Zeit, in der die Einspritzdüse offen ist, zu der Gesamtzeit definiert ist.

15. Verfahren nach Anspruch 14, **dadurch gekennzeichnet, dass** der Arbeitszyklus der Einspritzdüse in einem Bereich von 1 % bis 15 % variiert, vorzugsweise von 3 % bis 10 %, weiter vorzugsweise von 5 % bis 8 %.

16. Verfahren nach einem der Ansprüche 10 bis 15, **dadurch gekennzeichnet, dass** die temperaturregulierenden Parameter derart variieren, dass sich die Temperatur der Düse (7) von einer Anfangstemperatur im Bereich von 60 °C bis 100 °C, vorzugsweise von 70 °C bis 90 °C, weiter vorzugsweise 85 °C, auf eine Endtemperatur im Bereich von 0 °C bis 20 °C, vorzugsweise von 5 °C bis 15 °C, weiter vorzugsweise 10 °C bewegt.

17. Verfahren nach einem der Ansprüche 10 bis 16, **dadurch gekennzeichnet, dass** die Vorrichtung (1) weiterhin eine Batterie und einen Spannungsverstärker umfasst, und wobei das Steuerungsmodul (14) weiterhin die spannungsverstärkenden Parameter des Spannungsverstärkers reguliert, indem die spannungsregulierenden Parameter gemäß einer Funktion, die mit der Zeit steigt, erhöht werden.

18. Kosmetikproduktbehälter (5), der für die Verwendung in einer Vorrichtung (1) nach Anspruch 1 geeignet ist, umfassend einen Hauptkörper und eine Abdeckung aus Elastomer, wobei der Behälter (5) zum Beispiel ein Vial ist, und der eine Mischung aus einem Kosmetikprodukt und einem Alkohol enthält.

19. Kosmetikproduktbehälter (5) nach Anspruch 18, **dadurch gekennzeichnet, dass** die Menge des Alkohols weniger als 25 % in Bezug auf das Gesamtvolumen beträgt, vorzugsweise von 5 % bis 20 %, weiter vorzugsweise von 10 % bis 15 %.

## Revendications

1. Dispositif (1) destiné à l'administration d'un produit cosmétique, **caractérisé en ce qu'**il comprend:
- un premier support (2) destiné à une cartouche de gaz sous pression (3),
- un second support (4) destiné à un conteneur de produit cosmétique (5),
- un moyen de régulation de débit (6),
- une buse de sortie (7),
- un moyen de chauffage (8) de ladite buse (7),
- un déclencheur (9), et
- un module de commande (14),
dans lequel ledit premier support (2) est configuré pour loger une cartouche de gaz sous pression (3) destinée au passage d'un gaz sous pression vers ledit moyen de régulation de débit (6), ledit premier support (2), ledit moyen de régulation de débit (6) et ladite buse (7) étant reliés fluidiquement, de sorte que ledit moyen de régulation soit situé en aval dudit premier support (2) et en amont de ladite buse de sortie (7),
dans lequel ledit second support (4) est configuré pour loger un conteneur de produit cosmétique (5) destiné au passage d'un produit cosmétique vers ladite buse (7), et ledit second support (4) est relié fluidiquement à ladite buse (7) au moyen d'un premier conduit de sortie de produit (10),
et dans lequel ledit déclencheur (9) est configuré pour ouvrir le passage dudit gaz lorsqu'il est actionné par un utilisateur, et ledit module de commande (14) est configuré pour réguler les paramètres de régulation de débit dudit moyen de régulation de débit (6), et les paramètres de régulation de température dudit moyen de chauffage (8) de ladite buse (7).

2. Dispositif (1) selon la revendication 1, **caractérisé en ce que** ladite buse de sortie (7) est composée d'un matériau céramique.

3. Dispositif (1) selon l'une quelconque des revendications 1 ou 2, **caractérisé en ce qu'**il comprend en outre un premier moyen de fixation (12) destiné à fixer ladite cartouche de gaz sous pression (3) contre ledit moyen de régulation de débit (6), configuré de sorte que la fixation entre une sortie de ladite cartouche de gaz sous pression (3) et une entrée dudit moyen de régulation de débit (6) soit étanche.

4. Dispositif (1) selon l'une quelconque des revendications 1 à 3, **caractérisé en ce que** ledit moyen de régulation de débit (6) et ledit second support (4) sont en outre reliés fluidiquement au moyen d'un second conduit (11) qui s'étend jusqu'audit second support (4); et, de préférence, dans lequel n'importe lequel dudit premier conduit de sortie de produit (10) ou dudit second conduit d'entrée de gaz (11) possède un diamètre interne compris entre 0,4 mm et 1,2 mm, de préférence entre 0,5 mm et 1 mm, plus préférentement de 0,7 mm.

5. Dispositif (1) selon l'une quelconque des revendications 1 à 4, **caractérisé en ce que** ledit second support (4) comprend en outre un second moyen de fixation (12) destiné audit conteneur de produit cosmétique (5).

6. Dispositif (1) selon l'une quelconque des revendications 1 à 5, **caractérisé en ce que** ledit premier support (2) est configuré pour contenir une cartouche (3) qui contient du CO₂ à une pression de 50 bar.

7. Dispositif (1) selon l'une quelconque des revendications 1 à 6, **caractérisé en ce que** ledit second support (4) est configuré pour contenir un conteneur (5) qui contient une quantité de produit qui est comprise entre 2 et 6 cm³, de préférence entre 3 et 5 cm³, plus préférentement de 4 cm3.

8. Dispositif (1) selon l'une quelconque des revendications 1 à 7, **caractérisé en ce qu'**il comprend en outre un moyen d'alimentation électrique (13) et un régulateur de tension; dans lequel ledit moyen d'alimentation électrique (13) comprend de préférence une batterie qui présente une tension comprise entre 9 V et 15 V, plus préférentement entre 10 V et 12 V, plus préférentement de 11 V; et ledit régulateur de tension comprend de préférence un élévateur de tension configuré pour fournir une tension de sortie comprise entre 22 V et 26 V, de préférence de 24 V.

9. Dispositif (1) selon l'une quelconque des revendications 1 à 8, **caractérisé en ce que** ledit moyen de régulation de débit (6) comprend un injecteur, et lesdits paramètres de régulation de débit comprennent une valeur du cycle de fonctionnement dudit injecteur, ledit cycle de fonctionnement étant défini comme le rapport entre la durée pendant laquelle ledit injecteur est ouvert et la durée totale.

10. Processus d'administration d'un produit cosmétique qui, en partant d'un dispositif (1) selon la revendication 1, comprend les étapes suivantes:
[a] placer, par un utilisateur, une cartouche de gaz sous pression (3) dans ledit premier support (2) et un conteneur de produit cosmétique (5) dans ledit second support (4),
[b] actionner ledit déclencheur (9) par l'utilisateur,
[c] chauffer ladite buse (7) par ledit moyen de chauffage (8),
[d] ouvrir, par ledit déclencheur (9), le passage d'un gaz sous pression situé à l'intérieur de ladite cartouche de gaz sous pression (3),
[e] réguler, par ledit moyen de régulation de débit (6), le débit de sortie du gaz par ladite buse (7),
[f] dès qu'il sort, faire en sorte que ledit gaz provoque également la sortie d'un produit cosmétique situé à l'intérieur dudit conteneur de produit cosmétique (5), et
[g] mélanger le produit cosmétique avec le gaz dans ladite buse (7), et le pulvériser sur la peau d'un patient;
dans lequel ledit module de commande (14) prévu dans ledit dispositif (1) contrôle les paramètres de régulation de débit dudit moyen de régulation de débit (6) et les paramètres de régulation de température dudit moyen de chauffage (8) de la buse (7).

11. Processus selon la revendication 10, **caractérisé en ce qu'**il comprend en outre une étape préalable qui consiste **en ce que** ledit moyen de chauffage (8) chauffe ladite buse (7) à un moment qui précède l'étape [c].

12. Processus selon la revendication 11, **caractérisé en ce que**, au moment où le gaz commence à sortir par ladite buse (7), ledit moyen de chauffage (8) a chauffé ladite buse (7) à une température initiale comprise entre 60°C et 100°C, de préférence entre 70°C et 90°C, plus préférentement de 85°C.

13. Processus selon l'une quelconque des revendications 10 à 12, **caractérisé en ce qu'**une partie dudit gaz sous pression est introduite dans ledit conteneur de produit (5) par un second conduit d'entrée de gaz (11) et force ledit produit à sortir par ledit premier conduit de sortie de produit (10).

14. Processus selon l'une quelconque des revendications 10 à 13, **caractérisé en ce que** ledit moyen de régulation de débit (6) comprend un injecteur et lesdits paramètres de régulation de débit comprennent un cycle de fonctionnement dudit injecteur, ledit cycle de fonctionnement étant défini comme le rapport entre la durée pendant laquelle ledit injecteur est ouvert et la durée totale.

15. Processus selon la revendication 14, **caractérisé en ce que** ledit cycle de fonctionnement dudit injecteur varie sur une plage comprise entre 1 % et 15%, de préférence entre 3% et 10%, plus préférentement entre 5% et 8%.

16. Processus selon l'une quelconque des revendications 10 à 15, **caractérisé en ce que** lesdits paramètres de régulation de température varient de sorte que la température de ladite buse (7) passe d'une température initiale comprise entre 60°C et 100°C, de préférence entre 70°C et 90°C, plus préférentement de 85°C, à une température finale comprise entre 0°C et 20°C, de préférence entre 5°C et 15°C, plus préférentement de 10°C.

17. Processus selon l'une quelconque des revendications 10 à 16, **caractérisé en ce que** ledit dispositif (1) comprend en outre une batterie et un élévateur de tension, et ledit module de commande (14) régule en outre des paramètres d'élévation de tension dudit élévateur de tension en augmentant lesdits paramètres d'élévation de tension en suivant une fonction qui augmente au fil du temps.

18. Conteneur de produit cosmétique (5) adapté pour être utilisé dans un dispositif (1) selon la revendication 1, comprenant un corps principal et un couvercle, composé d'un élastomère, par exemple, ledit conteneur (5) étant de préférence un flacon, et conteneur un mélange de produit cosmétique avec un alcool.

19. Conteneur de produit cosmétique (5) selon la revendication 18, **caractérisé en ce que** la quantité dudit alcool est inférieure à 25% par rapport au volume total, de préférence entre 5% et 20%, plus préférentement entre 10% et 15%.
